(19) Europäisches Patentamt
European Patent Office
Office européen des brevets

(11) Publication number: 0 243 192 B1

(12) **EUROPEAN PATENT SPECIFICATION**

(45) Date of publication of patent specification: **15.07.92**

(51) Int. Cl.[5]: **A61K 31/52**

(21) Application number: **87303625.5**

(22) Date of filing: **24.04.87**

The file contains technical information submitted after the application was filed and not included in this specification

# (54) Antiviral 8-Phenylxanthines.

(30) Priority: **25.04.86 GB 8610136**

(43) Date of publication of application: **28.10.87 Bulletin 87/44**

(45) Publication of the grant of the patent: **15.07.92 Bulletin 92/29**

(84) Designated Contracting States: **BE CH DE FR GB IT LI LU NL SE**

(56) References cited:
**EP-A- 203 721**

**ANTIVIRAL RESEARCH, suppl. 1, 1985, pages 11-19, Elsevier Science Publishers B.V.; E. DE CLERCQ: "Recent trends and development in antiviral chemotherapy"**

(73) Proprietor: **THE WELLCOME FOUNDATION LIMITED**
**Unicorn House 160 Euston Road**
**London NW1 2BP(GB)**

(72) Inventor: **Lehrman, Sandra Nusinoff**
**2720 Old Sugar Road**
**Durham North Carolina 27707(US)**
Inventor: **Daluge, Susan Mary**
**297 Azalea Drive**
**Chapel Hill North Carolina 27514(US)**

(74) Representative: **Garrett, Michael et al**
**The Wellcome Research Laboratories Group Patents and Agreements Langley Court**
**Beckenham Kent BR3 3BS(GB)**

EP 0 243 192 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to certain 8-phenyl xanthine derivatives and salts and solvates thereof, and more particularly to their use in medicine for the treatment or prophylaxis of viral infections, particularly retroviral infections.

In the field of antiviral chemotherapy, relatively few drugs have been discovered which effectively combat the virus per se, owing to the difficulty of attacking the virus while leaving uninfected host cells unimpaired. It has recently been established that certain stages in the virus life-cycle, which vary from species to species, are specified by the virus itself. These stages may prove susceptible to attack where they differ sufficiently from any corresponding host-cell function. However, owing to great similarity between viral and host functions, effective treatments have proven very difficult to identify.

One group of viruses which has recently assumed a particular importance are the retroviruses. Retroviruses form a sub-group of RNA viruses which, in order to replicate, must first 'reverse transcribe' the RNA of their genome into DNA ('transcription' conventionally describes the synthesis of RNA from DNA). Once in the form of DNA, the viral genome is incorporated into the host cell genome, allowing it to take full advantage of the host cell's transcription/translation machinery for the purposes of replication. Once incorporated, the viral DNA is virtually indistinguishable from the host's DNA and, in this state, the virus may persist for as long as the cell lives. As it is virtually invulnerable to attack in this form, any treatment must be directed at another stage of the virus life cycle and will, of necessity, have to be continued until all virus-infected cells have died.

A species of retrovirus has also been reproducibly isolated from patients with Acquired Immune Deficiency Syndrome (AIDS). The virus has previously been referred to as human T-cell lymphotropic virus III (HTLV III), AIDS associated retrovirus (ARV), or lymphadenopathy associated virus (LAV), but is known internationally as human immunodeficiency virus (HIV) (Nature, Vol 321 1st May 1986). This virus (referred to herein as HIV) has been shown preferentially to infect and destroy T-cells bearing the $OKT^4$ surface marker and is now generally accepted as the aetiologic agent of AIDS. The patient progressively loses this set of T-cells, upsetting the overall balance of the immune system, reducing his ability to combat other infections, and predisposing him to opportunistic infections which frequently prove fatal. Thus, the usual cause of death in AIDS victims is by opportunistic infection, such as pneumonia or virally induced cancers, and not as a direct result of HIV infection. Other conditions associated with HIV infection include thrombocytopaenia purpura and Kaposis sarcoma.

Recently, HIV has also been recovered from other tissue types, including B-cells expressing the $T^4$ marker, macrophages and non-blood associated tissue in the central nervous system. This infection of the central nervous system is not necessarily associated with classical AIDS and has been discovered in patients with asymptomatic HIV infections. HIV infection of the CNS is associated with progressive demyelination, leading to wasting and such symptoms as encephalopathy, progressive dysarthria, ataxia and disorientation Peripheral Neuropathy may also be associated with HIV infection. Further conditions associated with HIV infection are the asymptomatic carrier state, progressive generalised lymphadenopathy (PGL) and AIDS-related complex (ARC).

The existence of these human retroviruses and others has only recently been recognised and, as the diseases with which they are linked are of a life-threatening nature, there exists an urgent need to develop ways to combat these viruses.

De Clercq (Antiviral Research (1985), Suppl. 1, pp11-19) reports on the trends and developments in antiviral chemotherapy, focusing on selective antiherpetic agents and broad spectrum antiviral agents. However, there is no teaching or disclosure of the use of the compounds of formula (I) below for the treatment or prophylaxis of an HIV or any other viral infection.

In European Patent Specification EP-A-0 203721 there is described and claimed a class of 8-phenylxanthine derivatives that are described as being useful in medical therapy, particularly for the treatment or propylaxis of pathophysiological conditions arising from the cell-surface effects of adenosine.

We have now discovered that certain of the 8-phenylxanthine compounds, referred to in the above-mentioned European Patent Specification possess antiviral activity, particularly against retroviruses, especially HIV.

Accordingly, the present invention provides a compound of formula (I):

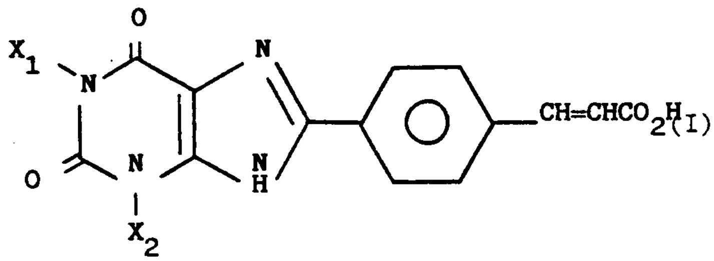

(I)

wherein:

$X_1$ and $X_2$ are the same or different and are hydrogen, $C_{1-6}$ alkyl, $C_{2-6}$ alkenyl or $C_{7-12}$ aralkyl provided that both $X_1$ and $X_2$ are not hydrogen; or a pharmaceutically acceptable salt, ester or solvate thereof, for use in the treatment or prophylaxis of a viral infection, especially a retroviral infection and particularly an HIV infection.

The compounds of formula (I) and their above salts, esters and solvates are hereinafter referred to as compounds of the invention. The compounds of the invention may exist in a number of tautomeric forms and all such forms, individually and as mixtures, are embraced by the above definition of formula (I) even though only one tautomer is depicted for convenience.

A preferred sub-class of $X_1$ and $X_2$, when $C_{7-12}$ aralkyl is benzyl.

Examples of $X_1$ and $X_2$, when $C_{1-6}$ alkyl, include both branched and straight chain alkyl, for example methyl, ethyl, n- and iso- propyl, and n-, iso- and tert- butyl. A preferred example of $X_1$ and $X_2$, when $C_{1-6}$ alkyl, is n-butyl.

Those compounds of formula (I) wherein $X_1$ and $X_2$ are the same or different and are $C_{7-12}$ aralkyl (e.g. benzyl) or $C_{3-4}$ straight chain alkyl, have been found to have particularly good anti-retroviral activity, especially against HIV.

The compounds of the present invention are capable of existing as geometric and optical isomers. All such isomers, individually and as mixtures, are included within the scope of the present invention. Compounds in the form of the E- geometrical isomers, are particularly preferred.

Of the compounds exemplified hereinafter, those that are preferred include

1. (E)-4-(1,3-dibutyl-1,2,3,6-tetrahydro-1,6-dioxo-9H-purin-8-yl)cinnamic acid,
2. (E)-4-(1,3-dibenzyl-1,2,3,6-tetrahydro-2,6-dioxo-9H-purin-8-yl)cinnamic acid,
3. (E)-4-(1-benzyl-1,2,3,6-tetrahydro-2,6-dioxo-3-propyl-9H-purin-8-yl)cinnamic acid,
4. (E)-4-(3-benzyl-1,2,3,6-tetrahydro-2,6-dioxo-1-propyl-9H-purin-8-yl)cinnamic acid.
5. (E)-4-(1-benzyl-1,2,3,6-tetrahydro-2,6-dioxo-3-butyl-9H-purin-8-yl)cinnamic acid,
6. (E)-4-(3-benzyl-1,2,3,6-tetrahydro-2,6-dioxo-1-butyl-9H-purin-8-yl)cinnamic acid.

Compound 2 is especially preferred on account of its particularly potent anti-HIV activity.

Examples of salts of a compound of formula (I) include ammonium salts, alkali metal salts, such as sodium and potassium salts, alkaline earth salts, such as magnesium and calcium salts, and salts formed with organic bases, for example, amino salts derived from mono-, di- or tri-(lower alkyl) or (lower alkanol)-amines, such as triethanolamine and diethylaminoethylamine, and salts with heterocyclic amines such as piperidine, pyridine, piperazine and morpholine. The salts have utility in the isolation and/or the purification of the compounds of the invention, and pharmaceutically unacceptable salts are also useful in being convertible to the acceptable salts by techniques well known the art.

Examples of esters according to the invention include alkyl (e.g. $C_{1-4}$ alkyl) or aryl (e.g. phenyl) carboxylate esters.

Examples of a solvate of a compound of formula (I) or a salt thereof include an hydrate, for example the monohydrate.

The invention provides a medicament for the treatment or prophylaxis of a human or non-human animal virus, especially retrovirus infection, including HIV infection which comprises an effective amount of a compound according to the invention. The present invention provides the use of a compound of the invention in the manufacture of a medicament for the treatment or prophylaxis of a viral or retroviral infection, especially an HIV infection.

Examples of other human retroviral infections include those caused by HTLV-I (eg. HTLV-1-positive leukaemia and lymphoma). Examples of nonhuman animal retroviral infections include feline leukaemia virus. Examples of clinical conditions associated with HIV and other retrovirus infections which may be treated or prevented in accordance with the present invention include AIDS, AIDS-related couplex (ARC), progressive generalized lymphadenopathy (PGL), as well as chronic neurological conditions associated with

retroviruses such as multiple sclerosis, tropical spastic paraparesis. Subjects having anti-HIV antibodies or serum HIV antigens can also be treated in accordance with the inventions.

The animal requiring treatment or prophylaxis with a compound of the present invention is usually a human or non-human mammal.

The route by which the compound of the present invention, is administered to the animal may be oral, parenteral (including subcutaneous, intradermal, intramuscular, intravenous or rectal. If the compound of the present invention presented in the form of a pharmaceutical formulation, which, as mentioned hereinafter, is preferred, then the actual formulation employed will of course depend on the route of administration elected by the physician or veterinarian. For example, if oral administration is preferred, then the pharmaceutical formulation employed is, preferably, one which is suitable for such a route.

A therapeutically or prophylactically effective amount of a compound of the present invention, will depend upon a number of factors including, for example, the age and weight of the animal, the precise condition requiring treatment or prophylaxis and its severity, and the route of administration, and will ultimately be at the discretion of the attendant physician or veterinarian.

An effective amount of a compound of the present invention for the treatment or prophylaxis of a virus infection will generally be in the range of 3.0 to 200 mg per kilogram body weight of the patient per day, preferably in the range of 6 to 150 mg per kilogram body weight per day and most preferably in the range 15 to 100 mg per kilogram body weight per day. The desired dose is preferably presented as two, three four, five, six or more sub-doses administered at appropriate intervals throughout the day. These sub-doses may be administered in unit dosage forms, for example, containing 10 to 2000 mg, preferably 20 to 1500 mg, and most preferably 50 to 1000 mg of active ingredient per unit dosage form.

An effective amount of a pharmaceutically acceptable salt or solvate of a compound of the present invention may be determined as a proportion of the effective amount of the compound per se.

The compounds according to the invention may be employed alone or in conjunction with one or more other therapeutic agents for example other anti-HIV agents e.g. 3'azidonucleosides such as 3'azido-3'deoxythymidine, 2',3'-dideoxynucleosides such as 2',3'-dideoxyadenosine or 2',3'-dideoxycytidine, immunomodulators or similar agents such as interleukin-II, interferon, granulocyte macrophage colony stimulating factor, as well as agents that may be of value for the treatment of opportunistic infections such as acyclovir, ganciclovir, desciclovir, idoxuridine (IUDR) or trifluridine.

While it is possible for the compounds of the present invention to be administered alone it is preferred to present them in the form of a pharmaceutical formulation, which comprises a compound of the present invention, and a pharmaceutically acceptable carrier therefor.

The pharmaceutical formulation may optionally contain other therapeutic agents that may usefully be employed in conjunction with the compound of the present invention, such agents include those referred to above. The expression "pharmaceutically acceptable" as used herein in relation to the carrier is used in the sense of being compatible with the compound of the present invention, employed in the formulation and with any other therapeutic agent that may be present, and not being detrimental to the recipient thereof. The carrier itself may constitute one or more excipients conventionally used in the art of pharmacy that enable the compound of the present invention, and any other therapeutic agent that may be present, to be formulated as a pharmaceutical formulation.

The pharmaceutical formulations include those suitable for oral, parenteral (including subcutaneous, intradermal, intramuscular and intravenous) and rectal administration although the most suitable route will probably depend upon, for example, the precise nature and severity of the condition and the identity of the recipient. The formulations may conveniently be presented in unit dosage form and may be prepared by any of the methods well known in the art of pharmacy. All methods include the step of bringing into association the active ingredient, i.e. the compound of the present invention, with the carrier. In general the formulations are prepared by uniformly and intimately bringing into association the active ingredient with, a liquid carrier or, a finely divided solid carrier or both, and then, if necessary, forming the associated mixture into the desired formulation.

The pharmaceutical formulations of the present invention suitable for oral administration may be presented as discrete units, such as a capsule, cachet, tablet, or lozenge, each containing a predetermined amount of the active ingredient; as a powder or granules; as a solution or a suspension in an aqueous liquid or a non-aqueous liquid such as a syrup, elixir or a draught; or as an oil-in- water liquid emulsion or a water-in-oil liquid emulsion. The formulation may also be a bolus, electuary or paste. Capsules and tablets are preferably enteric-coated.

Generally, a tablet is the most convenient pharmaceutical formulation suitable for oral administration. A tablet may be made by compressing or moulding the tablets may be prepared by compressing in a suitable machine the active ingredient in a free-flowing form, such as a powder or granules, in admixture with, for

example, a binding agent, an inert diluent, a lubricating agent, a disintegrating agent and/or a surface active agent. Moulded tablets may be prepared by moulding in a suitable machine a mixture of the powdered active ingredient moistened with an inert liquid diluent. The tablets may optionally be coated or scored and may be formulated so as to provide slow or controlled release of the active ingredient.

The pharmaceutical formulations suitable for parenteral administration include aqueous and non-aqueous sterile injection solutions which may contain, for example, an anti-oxidant, a buffer, a bacteriostat and a solute which renders the composition isotonic with the blood of the recipient, and aqueous and non-aqueous sterile suspensions which may contain, for example, a suspending agent and a thickening agent. The formulations may be presented in unit-dose or multi-dose containers, for example sealed ampoules and vials, and may be stored in a freeze-dried (lyophilized) condition requiring only the addition of the sterile liquid carrier, for example water for injection, immediately prior to use. Extemporaneous injection solutions and suspensions may be prepared from sterile powders, granules and tablets of the kind previously described.

The pharmaceutical formulations suitable for rectal administration may be presented as a suppository containing, for example, cocoa butter and polyethylene glycol.

An advantage of the compounds of formula (I) and the pharmaceutically acceptable salts and solvates thereof is that they are generally water soluble and that, therefore, they are suitable for formulation as aqueous solutions. In contrast, a number of the various substituted 8-phenylxanthines of the prior art (Biochem. Phamac., 1981, 30, 325 to 353; and Proc. Nat. Acad. Sci., 1983, 80, 2077 to 2080) are not soluble in water or at least not sufficiently soluble so as to permit their formulation in this way.

Example 1:

| Injection Formulation | |
|---|---|
| Ingredient | Amount per Ampoule |
| Compound of the Invention | 10.0mg |
| Buffering Agent, q.s. | - |
| Water, q.s. | 1.0ml |

The compound of the invention is finely ground and dissolved in the water. The pH is adjusted to the proper value by the buffering agent, and the solution is filtered and sterilised by autoclaving before being sealed under sterile conditions in ampoules.

Example 2:

| Suppository Formulation | |
|---|---|
| Ingredient | Amount per Ampoule |
| Compound of the invention | 75.0mg |
| Cocoa Butter or Wecobee™ Base | 2.0g |
| (Wecobee is a trademark and is a hydrogenated fatty carboxylic acid). | |

The compound of the invention is finely ground and mixed with the melted Cocoa Butter or Wecobee™ base. It is then poured into moulds and allowed to cool to afford the suppositories.

Example 3:

| Syrup Formulation | |
|---|---|
| Ingredient | Amount per 5ml |
| Compound of the Invention | 35.0mg |
| Sucrose | 2.0mg |
| Methylparaben | 0.5mg |
| Sodium Benzoate | 0.5mg |
| Cherry Flavour q.s. | |
| Colouring q.s. | |
| Water q.s. to | 5.0ml |

Ethanol, sucrose, sodium benzoate, methylparaben, and flavouring are combined in 70% of the total batch quantity of water. Colouring and the compound of the invention are then dissolved in the remaining water, and finally the two solutions are mixed and clarified by filtration, thus affording a syrup.

Example 4:

| Tablet Formulation | |
|---|---|
| Ingredient | Amount per Tablet |
| Compound of the Invention | 75.0mg |
| Lactose | 110.0mg |
| Corn Stach, Pregelatinized | 2.5mg |
| Potato Starch | 12.0mg |
| Magnesium stearate | 0.5mg |

The compound of the invention is finely ground and intimately mixed with the powdered excipients, lactose, corn stach, potato starch and magnesium stearate. The formulation is then compressed to afford tablets.

Example 5:

| Capsule Formulation | |
|---|---|
| Ingredient | Amount per Capsule |
| Compound of the Invention | 75.0mg |
| Lactose | 400.0mg |
| Magnesium Stearate | 5.0mg |

The compound of the invention is finely ground and mixed with the powdered excipients, lactose, corn starch and stearic acid, and then packed into two part, gelatin capsules.

Toxicity Data

The toxicity of a representative number of compounds of the present invention was determined by using the standard $LD_{50}$ test. The compounds were administered ntraperitoneally to mice and the results are set forth below.

| Compound | $LD_{50}$(mg/kg) |
|---|---|
| (E)-4-(1,2,3,6-tetrahydro-3-methyl-2,6-dioxo-9H-purin-8-yl)cinnamic acid | >500 |
| (E)-4-(1,2,3,6-tetrahydro-3-isobutyl-2,6-dioxo-9H-purin-8-yl)cinnamic acid | >100 |
| (E)-4-(1,2,3,6-tetrahydro-3-isobutyl-1-methyl-2,6-dioxo-9H-purin-8-yl)cinnamic acid | >100 |
| (E)-4-(1,3-dibutyl-1,2,3,6-tetrahydro-2,6-dioxo-9H-purin-8-yl)cinaamic acid | >100 |
| (E)-4-(3-butyl-1,2,3,6-tetrahydro-1-methyl-2,6-dioxo-9H-purin-8-yl)cinnamic acid | >100 |
| (E)-4-(3-benzyl-1,2,3,6-tetrahydro-1-methyl-2,6-dioxo-9H-purin-8-yl)cinnamic acid | >100 |

Antiviral Testing

The anti-HIV activity of various compounds was tested using an in-vitro assay system as described in H Mitsuya et al; (Proc Natl. Acad.Sci. USA Vol 82 pp 7096-7100, October 1985).

From the information obtained the 50% effective dose ($ED_{50}$ value) was estimated and the results expressed as active (+) or very active (++). Effects were seen at various concentrations of the drug ranging from 3µM to 30µM.

| Compound | µM | $ED_{50}$ |
|---|---|---|
| (E)-4-(1,3-dibutyl-1,2,3,6-tetrahydro-2,6-dioxo-9H-purin-8-yl)cinnamic acid | 30 | + |
| (E)-4-(1-Benzyl-1,2,3,6-tetrahydro-2,6-dioxo-3-propyl-9H-purin-8-yl)cinnamic acid | 30 | + |
| (E)-4-(3-Benzyl-1,2,3,6-tetrahydro-2,6-dioxo-1-propyl-9H-purin-8-yl)cinnamic acid | 30 | + |
| (E)-4-(1,3-dibenzyl-1,2,3,6-tetrahydro-2,6-dioxo-9H-purin-8-yl)cinnamic acid | 3-15 | + + |

**Claims**

1. Use of a compound for formula (I):

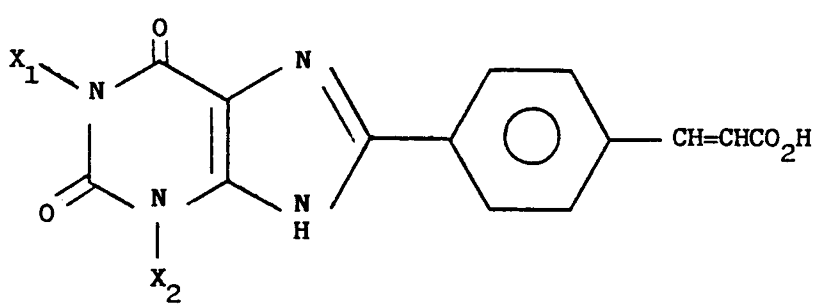

(I)

wherein:

$X_1$ and $X_2$ are the same or different and are hydrogen, $C_{1-6}$ alkyl, $C_{2-6}$ alkenyl or $C_{7-12}$ aralkyl optionally substituted in the aryl ring by $C_{1-6}$ alkyl provided that both $X_1$ and $X_2$ are not hydrogen; or a physiologically acceptable salt, ester or solvate thereof in the manufacture of a medicament for the treatment or prophylaxis of a viral infection.

2. Use of a compound of formula (I) (as claimed in claim 1) wherein said viral infection is a human retroviral infection.

3. Use of a compound of formula (I) (as claimed in claim 1) wherein said viral infection is an HIV infection.

4. Use of a compound of formula (I) (as claimed in claim 1) or a pharmaceutically acceptable salt, ester or solvate thereof, in the manufacture of a medicament for the treatment or prophylaxis of AIDS.

5. Use of a compound of formula (I) (as claimed in claim 1) or a pharmaceutically acceptable salt, ester or

solvate thereof, in the manufacture of a medicament for the treatment or prophylaxis of progressive generalised lymphadenopathy (PGL).

**6.** Use of a compound of formula (I) (as claimed in claim 1) or a pharmaceutically acceptable salt, ester or solvate thereof, in the manufacture of a medicament for the treatment or prophylaxis of AIDS related complex (ARC).

**7.** Use of a compound of formula (I) (as claimed in claim 1) or a pharmaceutically acceptable salt or solvate thereof, in the manufacture of a medicament for the treatment or prophylaxis of a HTLV-I infection.

**8.** Use of a compound of formula (I) (as claimed in claim 1) or a pharmaceutically acceptable salt or solvate thereof, in the manufacture of a medicament for the treatment or prophylaxis of the HIV carrier state.

**9.** Use of a compound of formula (I) (as claimed in claim 1) or a pharmaceutically acceptable salt or solvate thereof, in the manufacture of a medicament for the treatment or prophylaxis of a human subject having anti-HIV antibodies.

**10.** Use of a compound of formula (I) (as claimed in claim 1) or a pharmaceutically acceptable salt or solvate thereof, in the manufacture of a medicament for the treatment or prophylaxis of neurological disorders associated with human retroviral infections.

**11.** Use of a compound of formula (I) (as claimed in claim 1) where the compound is (E)-4-(1,3-dibenzyl-1,2,3,6-tetrahydro-2,6-dioxo- 9H-purin-8-yl) cinnamic acid or its salts in the manufacture of a medicament for the treatment or prophylaxis of a viral infection or any of the conditions as in claims 2 to 10.

**12.** Use of the compound as claimed in claim 11 wherein said salts are ammonium salts, alkali metal salts, alkaline earth salts or salts formed with organic bases.

**Revendications**

**1.** Utilisation d'un composé de la formule (I) :

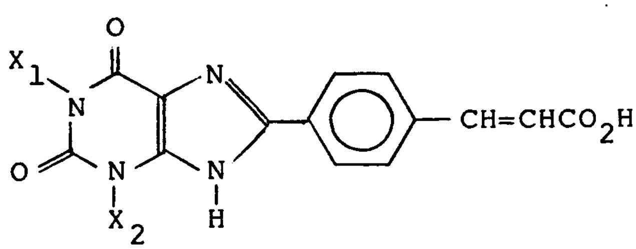

**(I)**

où $X_1$ et $X_2$ sont identiques ou différents et sont hydrogène, alkyle en $C_{1-6}$, alkényle en $C_{2-6}$ ou aralkyle en $c_{7-12}$ facultativement substitué dans le cycle aryle par alkyle en $C_{1-6}$, avec la restriction que $X_1$ et $X_2$ ne sont pas simultanément hydrogène;
ou d'un sel ester ou solvate physiologiquement acceptable de celui-ci dans la fabrication d'un médicament pour le traitement ou la prophylaxie d'une infection virale.

**2.** Utilisation d'un composé de la formule (I) (suivant la revendication 1), dans laquelle l'infection virale est une infection rétrovirale humaine.

**3.** Utilisation d'un composé de la formule (I) (suivant la revendication 1), dans laquelle l'infection virale est une infection à HIV.

**4.** Utilisation d'un composé de la formule (I) (suivant la revendication 1), ou d'un sel, ester ou solvate pharmaceutiquement acceptable de celui-ci, dans la fabrication d'un médicament pour le traitement ou

la prophylaxie du SIDA.

**5.** Utilisation d'un composé de la formule (I) (suivant la revendication 1) ou d'un sel, ester ou solvate pharmaceutiquement acceptable de celui-ci, dans la fabrication d'un médicament pour le traitement ou la prophylaxie de la lymphadépathie progressive généralisée (LPG).

**6.** Utilisation d'un composé de la formule (I) (suivant la revendication 1) ou d'un sel, ester ou solvate pharmaceutiquement acceptable de celui-ci, dans la fabrication d'un médicament pour le traitement ou la prophylaxie du complexe assimilé au SIDA.

**7.** Utilisation d'un composé de la formule (I) (suivant la revendication 1), ou d'un sel ou solvate pharmaceutiquement acceptable de celui-ci, dans la fabrication d'un médicament pour le traitement ou la prophylaxie d'une infection à HTLV I.

**8.** Utilisation d'un composé de la formule (I) (suivant la revendication 1) ou d'un sel ou solvate pharmaceutiquement acceptable de celui-ci, dans la fabrication d'un médicament pour le traitement et la prophylaxie de l'état de porteur de l'HIV.

**9.** Utilisation d'un composé de la formule (I) (suivant la revendication 1) ou d'un sel ou solvate pharmaceutiquement acceptable de celui-ci, dans la fabrication d'un médicament pour le traitement ou la prophylaxie chez l'être humain ayant des anticorps anti-HIV.

**10.** Utilisation d'un composé de la formule (I) (suivant la revendication 1) ou d'un sel ou solvate pharmaceutiquement acceptable de celui-ci, dans la fabrication d'un médicament pour le traitement ou la prophylaxie des désordres neurologiques associés aux infections rétrovirales humaines.

**11.** Utilisation d'un composé de la formule (I) (suivant la revendication 1), dans laquelle le composé est l'acide (E)-4-(1,3-dibenzyl-1,2,3,6-tétrahydro-2,6-dioxo-9H purin-8-yl)cinnamique et ses sels, dans la fabrication d'un médicament pour le traitement ou la prophylaxie d'une infection virale ou de tout autre état spécifié dans les revendications 2 à 10.

**12.** Utilisation du composé suivant la revendication 11, dans laquelle les sels sont les sels d'ammonium, les sels des métaux alcalins, les sels de métaux alcalino-terreux ou les sels formés avec les bases organiques.

**Patentansprüche**

**1.** Verwendung einer Verbindung der Formel (I)

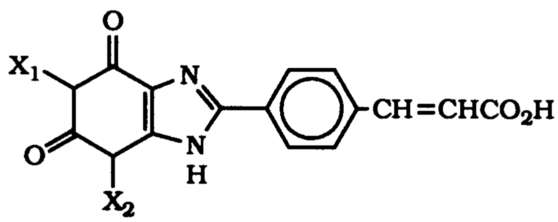

(I)

in der
$X_1$ und $X_2$ gleichartig oder verschieden sind und Wasserstoff, $C_{1-6}$-Alkyl, $C_{2-6}$-Alkenyl oder $C_{7-2}$-Aralkyl, gegebenenfalls am Arylring durch $C_{1-6}$-Alkyl substituiert, mit der Maßgabe bedeuten, daß $X_1$ und $X_2$ nicht gleichzeitig Wasserstoff bedeuten; oder ein physiologisch annehmbares Salz, ein Ester oder ein Solvat davon bei der Herstellung eines Arzneimittels zur Behandlung oder Prophylaxe einer Virusinfektion.

**2.** Verwendung einer Verbindung der Formel (I) (wie in Anspruch 1 beansprucht), worin die Virusinfektion eine Human-Retrovirusinfektion ist.

3. Verwendung einer Verbindung der Formel (I) (wie in Anspruch 1 beansprucht), worin die Virusinfektion eine HIV-Infektion ist.

4. Verwendung einer Verbindung der Formel (I) (wie in Anspruch 1 beansprucht) oder eines pharmazeutisch annehmbaren Salzes, Esters oder Solvats davon bei der Herstellung eines Arzneimittels zur Behandlung oder Prophylaxe von AIDS.

5. Verwendung einer Verbindung der Formel (I) (wie in Anspruch 1 beansprucht) oder eines pharmazeutisch annehmbaren Salzes, Esters oder Solvats davon bei der Herstellung eines Arzneimittels zur Behandlung oder Prophylaxe von progressiver allgemeiner Lymphadenopathie (PGL).

6. Verwendung einer Verbindung der Formel (I) (wie in Anspruch 1 beansprucht) oder eines pharmazeutisch annehmbaren Salzes, Esters oder Solvats davon bei der Herstellung eines Arzneimittels zur Behandlung oder Prophylaxe des AIDS-bedingten Komplexes (ARC).

7. Verwendung einer Verbindung der Formel (I) (wie in Anspruch 1 beansprucht) oder eines pharmazeutisch annehmbaren Salzes oder Solvats davon bei der Herstellung eines Arzneimittels zur Behandlung oder Prophylaxe einer HTLV-I-Infektion.

8. Verwendung einer Verbindung der Formel (I) (wie in Anspruch 1 beansprucht) oder eines pharmazeutisch annehmbaren Salzes oder Solvats davon bei der Herstellung eines Arzneimittels zur Behandlung oder Prophylaxe des HIV-Trägerzustands.

9. Verwendung einer Verbindung der Formel (I) (wie in Anspruch 1 beansprucht) oder eines pharmazeutisch annehmbaren Salzes oder Solvats davon bei der Herstellung eines Arzneimittels zur Behandlung oder Prophylaxe eines Anti-HIV-Antikörper aufweisenden Menschen.

10. Verwendung einer Verbindung der Formel (I) (wie in Anspruch 1 beansprucht) oder eines pharmazeutisch annehmbaren Salzes oder Solvats davon bei der Herstellung eines Arzneimittels zur Behandlung oder Prophylaxe von neurologischen Störungen, die durch Human-Retrovirusinfektionen bedingt sind.

11. Verwendung einer Verbindung der Formel (I) (wie in Anspruch 1 beansprucht), nämlich von (E)-4-(1,3-Dibenzyl-1,2,3,6-tetrahydro-2,6-dioxo-9H-purin-8-yl)-zimtsäure oder dessen Salze bei der Herstellung eines Arzneimittels zur Behandlung oder Prophylaxe einer Virusinfektion oder eines der Zustände gemäß einem der Ansprüche 2 bis 10.

12. Verwendung der Verbindung nach Anspruch 11, worin die Salze Ammoniumsalze, Alkalimetallsalze, Erdalkalimetallsalze oder mit organischen Basen gebildete Salze sind.